Europäisches Patentamt

European Patent Office

Office européen des brevets

(11) Publication number: **0 380 370 A2**

## EUROPEAN PATENT APPLICATION

(21) Application number: **90300861.3**

(22) Date of filing: **26.01.90**

(51) Int. Cl.5: **A61K 31/395, A61K 37/02, A61K 37/48, A61K 37/54, A61K 31/00**

(30) Priority: **26.01.89 US 302562**

(43) Date of publication of application:
**01.08.90 Bulletin 90/31**

(84) Designated Contracting States:
**AT BE CH DE DK ES FR GB GR IT LI LU NL SE**

(71) Applicant: **The Children's Medical Center Corporation**
**55 Shattuck Street**
**Boston, Massachusetts 02115(US)**

(72) Inventor: **Whalen, Giles F.**
**450 East 63rd Street, Apt. 7G**
**New York, New York 10021(US)**
Inventor: **Ingber, Donald E.**
**71 Montgomery Street, Boston**
**Massachusetts 02116(US)**

(74) Representative: **Deans, Michael John Percy et al**
**Lloyd Wise, Tregear & CO. Norman House**
**105-109 Strand**
**London WC2R OAE(GB)**

(54) **Inhibiting tumour recurrence after surgical resection.**

(57) To inhibit recurrence of a tumour at a local site in a mammal in conjunction with the resection of tissue at the site, there is applied to the site a composition which includes an agent for inhibiting attachment of a tumour cell in the area of the tissue.

EP 0 380 370 A2

## INHIBITING TUMOUR RECURRENCE AFTER SURGICAL RESECTION

This invention relates to preventing the recurrence of tumors at a local site after resection of tissue at that site.

Arons et al., 14 Cancer 1041, 1961, hypothesize that local recurrence of tumors may result from seeding of cancer cells in operative wounds. However, they found no correlation between the washing of such wounds with the cytotoxin 0.5% formaldehyde and local recurrence, distant metastasis, or survival of the treated animal.

McDonald et al., 80 A.M.A. Archives of Surgery 60, 1960, found that mechanical flushing of a wound with physiologic saline or distilled water is not sufficient to prevent seeding of cancer cells. However, they found that two percent solutions of the cytotoxins monoxychlorosene and mechlorethamine (nitrogen mustard) are effective in preventing tumor growth in laboratory animals. The use of these chemicals also delayed the wound healing process, and manifest highly toxic effects both locally and systemically. Such chemical treatment could be performed simultaneously with systemic treatment of bone marrow depressing agents. The authors found that a one percent solution of monoxychlorosene was toxic to tissues but ineffective in preventing tumor growth in experimental animals. They also found that 0.5% sodium hydrochlorite solution buffered to pH 9 was as effective as the above chemicals in preventing tumor growth in a laboratory animal, and considerably less toxic to the animal. They provide the following instruction: "[i]n choosing the agent to use as a wound irrigant, the surgeon must consider the relative effectiveness of the agent in killing cancer cells as well as the danger of systemic toxicity and local toxicity which could result in damage to normal tissues in the irrigated wound." They sought "to find a solution which could be used for wound irrigation following cancer surgery but which would not depress the function of the bone marrow".

In general, the invention features inhibiting recurrence of a tumor at a local site in a mammal in conjunction with resection of tissue at the site. The method includes the steps of resecting an area of tissue from the site, and applying to the site a composition (preferably a solution) which includes an agent for inhibiting attachment of a tumor cell in the area of the tissue. By "local site" is meant the site at which resection of tissue is performed in which a tumor is located, or sites at which surgical instruments used for the resection are contacted with tissue. By "inhibiting attachment of a tumor cell" is meant that an agent acts on components of a tumor cell involved in the process of attachment of the cell to the extracecellular matrix of the tissue, and does not directly kill the tumor cell, or inhibit intracellular metabolism, or DNA synthesis within the tumor cell. The agent acts by inhibiting formation of or by destabilizing adhesive contacts between a tumor cell surface and extracellular attachment molecules within a surgical wound. The inhibition of attachment may of course lead to eventual death of a tumor cell because that tumor cell is unable to obtain nourishment, or is more susceptible to attack by a mammal's immune defense system, e.g., cytotoxic immune cells, such as LAK cells, natural killer cells, and macrophages within the mammal. By "attachment" is meant the process by which a tumor cell anchors or fixes itself to an insoluble extracellular matrix, and thereby is able to maintain cell viability, stimulate vascularization, obtain nourishment, and proliferate in a sustained fashion.

In preferred arrangements, we apply a composition to the local site, including an agent which does not kill a tumor cell, inhibits the attachment of a tumor cell in the area of the tissue, and is relatively non-toxic to cells in the area of the tissue; most preferably the agent is chosen from compounds that modulate extracellular matrix metabolisn, e.g., compounds that inhibit deposition of extracellular matrix molecules that mediate tumor cell attachment, a proteolytic enzyme, e.g., that degrades the matrix molecules, a high molecular weight polymer, e.g., that can physically restrict access of a tumor cell to an attachment molecule within the tissue, and synthetic peptides or saccharides including a cell binding sequence of a specific extracellular matrix molecule, e.g., which competitively interfere with binding of a matrix molecule to a tumor cell surface receptor; even more preferably the agent is chosen from L-azetidine-2-carboxylic acid, Dispase (a bacterial neutral proteolytic enzyme), and a synthetic fibronectin peptide containing an arginine-glycine-aspartate (RGD) sequence which competitively inhibits tumor cell binding to an extracellular matrix.

The above method provides a non-toxic means for reducing the incidence of tumor cell attachment at the time of surgery. Applicants have recognized that non-toxic reversible inhibitors of tumor cell attachment provide a useful improvement to the surgical result by inhibiting tumor cell attachment long enough to reduce the incidence of tumor recurrence in a surgical wound. This method can be used when a tumor is resected, or even when a tissue is resected which is not known to contain a tumor, since use of the method entails little or no risk to the patient, but provides significant positive value in the event that tumor cells are present during a resection. The claimed agents for use in the method are advantageous because they do not cause side effects such as nausea, compromised immune response, or hair loss.

Other features and advantages will be apparent from the following description of preferred embodiments .

Attachment Inhibiting Agent

Preferred attachment inhibiting agents can be chosen by one of the assays described below from those agents or combinations of agents thought to inhibit cell attachment, which fall within the following four categrories of compounds: modulators of extracellular matrix metabolism, degradative enzymes, high molecular weight compounds that produce high medium viscosity, and agents which act on cell surfaces and interfere with binding to extracellular molecules or other cell surfaces. These categories of candidate compounds to be assayed will now be briefly discussed.

A modulator of extracellular matrix metabolism is a compound which affects the rate of degradation, or reduces the extent of extracellular matrix, prevents or reduces deposition of compounds of the matrix, or affects the integrity of the matrix scaffold within a mammal. Examples of such modulators include heparin, hydrocortisone-21-phosphate disodium salt, 4-pregnene-17-$\alpha$-21-diol-3,20-dione (cortexolone), inhibitors of protein synthesis, for example, cycloheximide, and specific inhibitors of collagen deposition, for example, the proline analog L-azetidine-2-carboxylic acid (LACA). Other examples include $\alpha$-, $\beta$- and $\gamma$-cyclodextrin sulfate, dextran sulfate and $\beta$-1,3-glucan sulfate. Combinations of these compounds may also be used, for example, the combination of heparin with angiostatic steroids such as hydrocortisone or cortexolone.

Enzymes which are commonly used to disrupt cell adhesive contacts during passaging of cultured cells are useful. Such enzymes include general proteases, such as trypsin, with or without a calcium chelator, such as EDTA, and the bacterial netural protease, Dispase. Other examples include enzymes derived from animals, plants or microorganisms including pancreatin (Gibco), collagenase from Chlostridium histolyticum, (Sigma), collagenase from Achromobacter iophagus - (Boehringer Mannheim), thermolysin from Bacillus thermoproteolyticus rokko (Sigma); thrombin from human plasma (Sigma); RDBTM from the Israel Institute, Funakosi; and enzymes which inhibit cell adhesion by decomposition of polysaccharides or oligosaccharides in an extracellular matrix or on a cell surface, e.g., general glycosidases, such as heparinase, sialidase and mannosidase.

High molecular weight compounds includes those which can be used to physically interfere with tumor cell attachment by altering the viscosity of the medium around those cells. Examples of such polymers include dextran, of molecular weight 10,000 or greater, agarose, and polyethylene oxide of molecular weight 35,000 to 100,000, arabic gum, other polysaccharides of microbial origin and derivatives thereof, dextran sulfate, $\beta$-1,3-glucan (cardran), $\beta$-1,3-glucan sulfate, xanthan gum, methyl cellulose, carboxymethyl cellulose, polyvinyl compounds such as polyvinyl alcohol and polyvinyl pyrrolidone, and polycarboxylic acids such as polyacrylic acid, and polyethylene glycol.

Agents which specifically interfere with binding of a cell surface receptor with specific molecules, within the extracellular matrix or milieu, that can mediate tumor cell attachment may also be used. Such agents specifically interfere with the binding, for example, of fibronectin to cell surface receptors of tissue cells. Examples of these compounds include small synthetic peptides, such as glycine-arginine-glycine-aspartate-serine-proline (GRGDSP), which contain an RGD sequence which competitively inhibits the cell-specific binding activity of fibronectin. A related peptide, containing a single amino acid substitution, may also be suitable for some tumor cell types, for example, glycine-arginine-glycine-glutamate-serine-proline (GRGESP) can be used with some melanoma cells. Other examples include the peptides RGDS and GRGDS. Cell binding peptides from other extracellular matrix molecules, e.g., laminin proteoglycans or types of collagens, as well as other types of proteases are suitable. These peptides contain a YIGSR sequence and include, for example, YIGSR and CDPYIGSR. Additional agents of this class include lectins, such as conconavalin A, or soluble saccharide moieties responsible for specific cell interactions with glycosaminoglycans and glycoproteins, for example, heparin fragments or soluble mono- and oligo-saccharides. Cyclic peptides containing the RGD sequence, such as Gly-Pen-Gly-Arg-Gly-Asp-Ser-Pro-Cys-Ala, where Pen is penicillamine, may also be used.

The above agents may all be useful.

The in vivo or in vitro tests described below provide a simple means to determine whether a particular agent is useful. Other tests also can be used which are designed to determine whether the agent is able to prevent attachment of a tumor cell to an underlying structure that is analogous to those found within a surgical wound.

In Vitro Assay

The following two examples of assays for suitable agents are designed to approximate a wound environment, and thus act as quick tests for suitable agents for practical use in particular circumstances. These assays are not limiting to the application. One example of an in vitro assay for determining the effect of one of the above agents on tumor cell attachment was performed as follows. Gelatinized 96-well tissue culture plates (Co-star, Cambridge, MA) were prepared by treating a standard culture plate with 100 microliters of 1.5% gelatin (Difco, Detroit, MI) in phosphate-buffered saline (PBS) and incubating the plate at 37°C for at least 24 hours. The solution was then aspirated and the cells washed twice with 200 microliters of PBS prior to use. Test agents were diluted in Dulbecco's Modified Eagles Medium (DMEM; Gibco Laboratories, Grand Island, NY) containing 10% fetal calf serum (FCS; Gibco), and 100 microliters of the diluted agent added to each well. An aliquot of a melanoma cell line, for example, B16-F10 (provided by Dr. Bruce Zetter, Children's Hospital, Boston, MA), including $10^4$ cells per 100 microliters culture medium was added to each well, and the plates incubated at 37°C for 4 or 16 hours. The cells were grown in DMEM supplemented with 10% FCS, 2 mM glutamine and 100 units per ml penicillin and streptomycin.

Other tumor cell lines may also be used, for example, Lewis lung carcinoma, reticulum cell sarcoma, and MB-49 bladder carcinoma. It is also possible to use a human tumor cell line isolated from an appropriate tissue of the patient undergoing surgery, or cells previously isolated from a tumor in the same patient during a prior resection. Generally, any cell line which is able to be cultured in vitro and to attach to the above gelatinized culture plates is suitable. Moreover, plates coated with other types of adhesive matrix molecules, such as laminin, fibronectin, fibrin, vitronectin, proteoglycans, or types IV or V collagens, may be used if the chosen tumor cell does not attach to gelatin. Complex extracellular matrices such as native collagen gels, basement membrane gels (e.g., Matrigel, EHS laminin gels), or fibrin clots may also be utilized as adhesive substrata in this assay.

After the cells and agent were incubated, the medium was aspirated from each well and the well washed twice with 200 microliters of PBS. The number of cells attached to each culture well was determined using an acid phosphatase colorimetric assay as described by Connaly et al., Analytical Biochemistry 152:146 (1986). Briefly, 100 microliters of an acid phosphatase reagent (0.1M sodium acetate, 0.01M p-nitrophenyl phosphate, 0.1% Triton-X 100) was added to each well, the plates incubated at 37°C for two hours, and 10 microliters 1N NaOH added to each well. The optical density of each well was read at 405nm on a Biotek ELISA reader. The optical density is directly related to cell number. The results were expressed as percent of optical density in untreated control wells, and represent the mean of at least three wells. Control values have a standard error of the mean of less than 10%.

Other standard methods can also be used for quantitation of the number of cells attached to a well. For example, a Coulter counter or a hemacytometer can be used, radioactively-labelled cells can be counted by standard technique, or standard DNA or protein assays, as well as other colorimetric assays calibrated to quantitate cell numbers can be used.

In order to determine whether inhibition of cell attachment was non-toxic and reversible with any particular agent, the tumor cells were incubated in the presence of the agent and unattached cells collected, washed twice with serum containing medium, and replated on gelatinized well plates in new culture medium. Control cells were handled in a similar manner, except that they were removed from wells 16 hours after incubation by trypsinization. After incubation for 4-16 hours, the medium was aspirated, the wells washed twice with 200 microliters of PBS, and the remaining attached cells trypsinized and counted with a Coulter counter.

In Vivo Test

Inhibitors of tumor cell attachment in vivo are tested by their ability to block tumor cell attachment in a surgical wound. Wounds were made on the backs of C57 B1/6 mice (Charles River Laboratories, Wilmington, MA) using aseptic procedures under methoxyflurane anesthesia (Pittman-Moore Inc., Washington Crossing, NJ). The wounds were made with scissors which were invisibly contaminated by B16-F10 melonoma by taking two snips of a tumor (approximately 500 mm³ growing subcutaneously on the back of a single animal). The scissors were used to create a lateral subcutaneous pocket off a dorsal mid-line wound in an uncontaminated animal. Prior to use, the scissors were inspected to insure that no visible tissue was present on them. Prior to closure, the wounds were vigorously irrigated with 3ml of Ringers lactate supplemented with a chosen agent. Excess fluid was then expressed and the wound closed with skin clips. After 21, 30, and 45 days following surgery the number of animals developing tumors was determined and the maximal length and width of the tumor measured using calipers.

Other methods that can be used for the in vivo assay include injection of tumor cells isolated from tumor cell cultures, or by passage in other tumor-bearing animals. Tumor cells may be administered using a syringe as a single cell suspension, as multicellular clumps, or as macroscopic tumor cell aggregates using a trochar.

The percent of animals which develop tumors after contamination in the above in vivo assay is dependent upon the type of tumor cell used in the inoculation procedure. For example, using the above cell lines, the occurrence of tumors range from 50-100%. B16-F10 melanoma produced 100% tumor formation.

Combination of heparin (100 micrograms per ml) and cortexolone ( 10-200 micrograms per ml) or hydrocortisone (12.5-200 micrograms per ml) produced some inhibition of tumor cell attachment in the in vitro system. LACA reversibly inhibited tumor cell attachment when administered in doses greater than 25 micrograms per ml. That is, cells that were prevented from attaching by exposure to LACA were able to be replated in fresh medium when transferred to new dishes. Dispase completely prevented tumor cell attachment after four hours and this effect was reversible. Agarose, at concentrations around 10 mg/ml, when the solution had a high viscosity, blocked attachment to some extent. The synthetic peptide GRGDSP reversibly inhibited tumor cell attachment at concentrations greater than 10 mg/ml. The peptide GRGESP also inhibited attachment of B16 melanoma cells but was less effective.

In the in vivo assay GRGDS and Dispase inhibited formation of a significant percentage of tumors in animals, and caused the average volume of tumors formed in other animals to be significantly smaller than in control animals. No evidence of infection from use of these agents was detected, nor was the wound healing process delayed.

Use of Attachment Inhibitor Agents

The above agents, which are determined to be inhibitory in the in vivo or the in vitro assay, are used at concentrations determined to be active in preventing tumor cell attachment without significantly affecting the viability of the tumor cells, and thus without being toxic to surrounding tissue cells. Typically, the agents are used as an irrigating solution at the time of surgery. This is done by pouring a solution into the operative field at the conclusion of resection, and allowing the solution to sit for a period of time sufficient to prevent attachment of tumor cells. The solution is then removed by draining, using a suction aspirator or other vacuum device.

In an alternative method, the operative field is forcefully irrigated with a solution using a syringe, or other jetting device, or a pressurized spray. The solutions may also be used in a continuous fashion by providing an irrigation catheter left in the operative field, or placed within a body cavity, such as the peritoneum or pleura.

The solutions may also be used during endoscopic procedures as continuous irrigants or intermittant irrigants. The inhibitory agents may also be applied as a semi-solid film over surfaces of tissues exposed during surgical resection.

Irrigating solutions of the present invention are useful after resection of any malignant tumor from any part of a mammalian body. In particular, they are useful whenever a tumor must be removed with a relatively close margin, such as in surgery of head and neck tumors. They are also useful after a mastectomy or lymph node dissection in the groin and axilla, the resection of a rectal carcinoma, and the conservative resection of a soft tissue carcinoma. Similarly, the method is useful where tumor cells have clearly been freed and spilled into a wound or body cavity, for example, when a cystic tumor is ruptured, or during resection of an ovarian tumor, or some renal tumors.

If it is known that a particular tumor is potentially able to spread from one location to another, an irrigating solution should be used. One example is when a bowel cancer perforates through the bowel wall and may be seeding the perentoneal cavity. In endoscopic surgery, implantation of pretumor cells can be a problem in transurethral resection or removal of small bladder tumors. During these procedures irrigation of the operative field should also be performed. In addition, even a benign tumor, such as polymorphic adenoma of the saliva glands or a pseudomyxomer peritonea of the ovary and appendix, have a propensity to implant in an operative field. Thus, inhibitors of such implantation will be useful to prevent benign tumors reoccurring.

Without being bound to any specific theory, applicants believe that the method of this invention is suitable for control of recurrence of tumors at a local site in a mammal because of inhibition of tumor cell attachment to the underlying extracellular matrix. Unattached tumor cells are thought to be more vulnerable to host immune responses, and are more easily displaced from a wound or serous cavity by irrigation. Prevention of attachment may cause the cell to loose its viability because of its inability to attach.

Other arrangements are feasible.

For example, immunostimulants can be applied at a local site simultaneously or separately with the above agents. Such application will enhance the effect of the agents. Examples of immunostimulants include low molecular weight immunostimulants, such as muramyldipeptide derivatives (e.g.

(muramyldipeptide)lysine, N-acetyl-6-O-[10-(2,3-dimethoxy-5-methyl-1,4-benzoquinon-6-yl)-decanoyl]muramyl-L-valyl-D-isoglutamine methyl ester (TMB-66)), trehalose dimycolate, treharose dicorynomycolate, bestatin; high molecular weight immunostimulants, e.g., Picibanil (OK-432, a lyophilized powder of avirulent Streptococcus pyogenes), Krestin (PSK, prepared from the mycelia of Coriolus versicolor), Shizophyllan (a glucan extracted from the fermentation broth of Shizophyllum commune), and Lentinan (a β-glucan extracted from Lentinus edodes); and immunostimulants of human origin, e.g., IFN-γ, IL-1α, IL-1β, IL-2, IL-3, IL-4, TNFα and GM-CSF.

**Claims**

1. A method for inhibiting recurrence of a tumor at a local site in a mammal in conjunction with resection of tissue at said site, said method comprising the steps of:

a) resecting an area of tissue from said site,

b) applying to said site a composition comprising an agent for inhibiting attachment of a tumor cell in the area of said tissue.

2. The method of claim 1 wherein said method comprises applying a composition comprising an agent which does not kill said tumor cell, inhibits said attachment of said tumor cell, and is relatively non-toxic to cells in the area of said tissue.

3. The method of claim 2 wherein said agent is chosen from a group consisting of (a) modulators of extracellular matrix metabolism, (b) proteolytic enzymes, (c) high molecular weight polymers, and (d) synthetic peptides or saccharides containing a cell binding sequence of a specific extracellular matrix molecule.

4. The method of claim 3 wherein said agent is chosen from the group consisting of (a) L-azetidine-2-carboxylic acid, (b) Dispase, and (c) a synthetic fibronectin peptide containing an arginine-glycine-aspartate sequence.

5. The method of claim 1, wherein said composition is applied to said mammal in combination with an immunostimulant.

6. The method of claim 5, where said immunostimulant and said composition are applied simultaneously to said mammal.

7. The method of claim 1 in which said agent comprises an enzyme which inhibits the attachment of said tumor cell.

8. The method of claim 7 in which said enzyme is capable of disrupting adhesive contacts of cells.

9. The method of claim 1 in which said agent comprises a proteolytic enzyme.

10. The method of Claim 1 in which said agent comprises dispase, trypsin, pancreatin, collagenase or thermolysin.

11. The method of Claim 1 in which said agent comprises a glycosidase, preferably heparinase, sialdase or mannosidase.

12. The method of Claim 1 in which said agent is a synthetic fibronectin peptide containing an arginine-glycine-aspartate sequence.

13. The method of Claim 7 in which said agent comprises the sequences GRGDS, GRGES, or Gly-Pen-Gly-Arg-Gly-Asp-Ser-Pro-Cys-Ala.

14. The method of Claim 1 in which said agent comprises GRGDSP, GRGESP, RGDS, GRGDS or Gly-Pen-Gly-Arg-Gly-Asp-Ser-Pro-Cys-Ala.

15. L-azetidine-2-carboxylic acid.

16. A synthetic fibronectin peptide containing an arginine-glycine-aspartate sequence.

17. An enzyme adapted operatively to inhibit recurrence of a tumor at a local site in a mammal in conjunction with resection of tissue at said site and comprising: dispase; trypsin; pancreatin; collagenase; a glycosidase, preferably heparinase, sialdase or mammosidase; or thermolysin; said enzyme being for use in surgery involving resection of tissue.

18. For use in the manufacture of a medicament for use in surgery involving resection of tissue at a local site in a mammal to inhibit recurrence of a tumour at said site, any of the agents identified in Claims 13 to 17.

19. An agent selected from modulators of extracellular matrix metabolism, degradative enzymes, high molecular weight compounds that produce high medium viscosity, and agents effective to act on cell surfaces and interfere with binding to extracellular molecules or other cell surfaces, all for use in the manufacture of a medicament for use in inhibiting recurrence of a tumour at a local site in a mammal in conjunction with resection of tissue at said site.

20. An agent as identified in any of Claims 13 to 16, for use in inhibiting recurrence of a tumour at a local site in a mammal in conjunction with resection of tissue at said site.